# EUROPEAN PATENT SPECIFICATION

(11) **EP 0 741 120 B1**
(45) Date of publication and mention of the grant of the patent: **26.03.2003**
(21) Application number: 96302884.0
(22) Date of filing: 25.04.1996
(51) Int. Cl.: C07C 9/22

(54) **Polyalphaolefin dimers having low kinematic viscosities**
Polyalphaolefin-Dimere mit niedrigen kinematischen Viskositäten
Dimers de poly-alpha-oléfine ayant des viscosités cinématiques faibles

(30) Priority: 05.05.1995 US 435935
(43) Date of publication of application: 06.11.1996
(73) Proprietor: Chevron Phillips Chemical Company LP, The Woodlands, TX 77380 (US)
(72) Inventor: Hope, Kenneth D., Kingwood, Texas 77339 (US); Ho, Ting C., Kingwood, Texas 77345 (US); Cupples, Barrett L., Kingwood, Texas 77345 (US)
(74) Representative: Nash, David Allan

(56) References cited:
- EP-A- 0 467 345
- GB-A- 2 024 846
- US-A- 5 420 373

## Description

The present invention relates to a process of producing dimers of olefins having improved low temperature quality.

### BACKGROUND OF THE INVENTION

It is well known to make polyalphaolefins by reacting 1-decene with boron trifluoride and butanol. The oligomer product is a mixture of dimer, trimer, and higher molecular weight materials. Dimers having good low temperature properties are useful in avionics coolants, dielectric fluids, and heat transfer fluids.

U.S. Patents 5,068,487; 5,171,905; and 5,171,918 disclose a process for producing predominately dimer and trimer. That process uses boron trifluoride in conjunction with alcohol alkoxylates

[RO(CHR'-CHR"O)ₙ-H].

Previously, we filed an application U.S. 08/217,265 to the use of boron trifluoride in conjunction with hydroxy carbonyls. In one embodiment of that invention, a ketone was used as a copromoter with the hydroxy carbonyl to improve dimer yield.

We also filed an application U.S. 08/298,635 to the use of boron trifluoride in conjunction with an alcohol alkoxylate and a ketone.

Other patents of interest are U.S. Patents 3,769,363: 3,997,621; 4,218,330; 4,436,947; and 4,982,026.

U.S. Patent 3,769,363 by Brennan discloses oligomerization of C₆-C₁₂ normal α-olefins, such as 1-decene, with boron trifluoride and C₅ carboxylic acid to improve trimer yields.

U.S. Patent 3,997,621 by Brennan discloses oligomerization of C₆₋C₁₂ normal α-olefins using alcohols or water promoters in conjunction with small amounts of methyl and ethyl esters of a C₂₋C₅ mono carboxylic acid to improve trimer yields.

U.S. Patent 4,218,330 by Shubkin discloses dimerization of a C₁₂₋C₁₈ monomer with a boron trifluoride-water complex and an excess of boron trifluoride. The product is distilled to remove the monomer and is hydrogenated for use as a crankcase lubricant. This product is mainly dimers, with minor amounts of trimers and higher oligomers.

U.S. Patent 4,436,947 by Morganson et al. discloses oligomerization of C₆-C₂₀ olefins, such as 1-decene, with boron trifluoride and a mixture of an aliphatic alcohol, an aliphatic ketone, and a polyol. This product is predominately trimer.

U.S. Patent 4,982,026 by Karn et al. discloses polymerization of lower alkene monomers (C₂₋C₆) with boron trifluoride and a strong acid, such as phosphoric acid, to produce a polymer having a molecular weight from 250 to 500 and having a high vinylidene content.

Other patents of interest are U.S. Patent Nos. 3,769,363: 3,997,621; 4,218,330; 4,436,947; 4,982,026 5,068,487; 5,171,905; and 5,171,918.

### SUMMARY OF THE INVENTION

We have identified what causes poor low temperature performance for hydrogenated dimers. The poor quality dimers have crystals that form at low temperatures. We cooled a poor quality dimer slowly until the crystals began to form, then separated the crystals from the dimer and analyzed the crystals.

Surprisingly, for the dimer 1-decene (C₁₀), the crystals were substantially normal eicosane (C₂₀), with lesser amounts of normal nonadecane (C₁₉) and normal octadecane (C₁₈), and trace amounts of normal heneicosane (C₂₁), normal heptadecane (C₁₇), normal docosane (C₂₂). When those crystals were dissolved in good dimer, the viscosity increased.

By adjusting operating parameters to reduce the presence of normal hexadecane (C₁₆) in 1-octene (C₈) dimer to less than 0.50 wt.%, we were able to achieve a -54° C viscosity of less than 280 cSt. By adjusting operating parameters to reduce the presence of normal eicosane in 1-decene dimer to less than 0.05 wt.%, we were able to achieve a -54° C viscosity of less than 1000 cSt and a -40° C viscosity of less than 240 cSt. By adjusting operating parameters to reduce the presence of normal tetracosane (C₂₄) in 1-dodecene dimer to less than 0.60 wt.%, we were able to achieve a -40° C viscosity of less than 1000 cSt. By adjusting operating parameters to reduce the presence of normal octacosane (C₂₈) in 1-tetradecene (C₁₄) dimer to less than 0.10 wt.%, we were able to achieve a -20° C viscosity of less than 1000 cSt.

In order to reduce the amounts of these normal paraffins, the amount of the normal paraffins and corresponding normal olefins in the feed to the hydrogenation unit should be limited. Prior to hydrogenation, a 1-octene dimer should have less than 0.50 wt.% normal C₁₆ materials, 1-decene dimer should have less than 0.05 wt.% normal C₂₀ materials, 1-dodecene dimer should have less than 0.60 wt.% normal C₂₂ materials, and a 1-tetradecene dimer should have less than 0.10 wt.% normal C₂₄ materials.

### BRIEF DESCRIPTION OF THE DRAWINGS

In order to assist the understanding of this invention, reference will now be made to the appended drawings. The drawings are exemplary only, and should not be construed as limiting the invention.

Figure 1 is a comparison of the gas chromatography results of the crystals discussed in Example 5 and a gas chromatography standard which contains straight chain saturated hydrocarbons. The peaks are labeled from C₁₇ to C₂₂, which correspond to heptadecane to docosane.

Figure 2 is a plot of the weight percent normal eicosane in the dimer versus the -54° C kinematic viscosity, which is also discussed in Example 5.

### DETAILED DESCRIPTION OF THE INVENTION

In its broadest aspect, the present invention involves achieving low temperature viscosities in oligomers by reducing the presence of certain normal paraffins to less than certain amounts in the dimer. For 1-octene dimer, the amount of normal hexadecane should be reduced to less than 0.50 wt.%. For 1-decene dimer, the amount of normal eicosane should be reduced to less than 0.05 wt.%. For 1-dodecene dimer, the amount of normal tetracosane should be reduced to less than 0.60 wt.%. For 1-tetradecene dimer, the amount of normal octacosane should be reduced to less than 0.10 wt.%.

The oligomerization of the olefinic monomer can be performed by contacting that monomer with boron trifluoride and a promoter, such as a hydroxy carbonyl promoter. The promoter can be used in conjunction with a secondary promoter.

### OLEFINIC MONOMER

Preferably, olefins used in making the oligomer are predominately (at least 50 mole %) C₆₋C₂₀ straight-chain, mono-olefinically unsaturated hydrocarbons in which the olefinic unsaturation occurs at the 1- or α-position of the straight carbon chain. Straight-chain α-olefins are preferred because they are more reactive and commercially available. Such α-olefins can be made by the thermal cracking of paraffinic hydrocarbons or by the well known Ziegler ethylene chain growth and displacement on triethyl aluminum. Individual olefins may be used, as well as mixtures of such olefins. Examples of such olefins are 1-hexene, 1-heptene, 1-octene, 1-nonene, 1-decene, 1-dodecene, 1-hexadecene and 1-tetradecene. The more preferred normal α-olefin monomers are those containing about 8 to 14 carbon atoms. The olefin monomers can also contain minor amounts of up to about 50 mole %, and usually less than 25 mole %, of internal olefins and vinylidene olefins.

### OLIGOMER PRODUCT

The oligomer product is that portion of the reaction product remaining after boron trifluoride, promoters, and unreacted monomer are removed. A 1-octene dimer should have less than 0.50 wt.% normal hexadecane to have a -54° C viscosity of less than 280 cSt. A 1-decene dimer should have less than 0.05 wt.% normal eicosane to have a -54° C viscosity of less than 1000 cSt and a -40° C viscosity of less than 240 cSt. A 1-dodecene dimer should have less than 0.60 wt.% normal tetracosane to have a -40° C viscosity of less than 1000 cSt. A 1-tetradecene dimer should have less than 0.10 wt.% normal octacosane to have a -20° C viscosity of less than 1000 cSt.

### OLIGOMERIZATION REACTION

The promoters are used in minor but effective amounts. For example, the total amount of promoters used can be from about 0.001 to 0.04 moles per mole of monomer (0.1 to 4.0 mole percent). In general, boron trifluoride is used in molar excess to the amount of promoter. This can be accomplished by using a closed reactor and maintaining a positive boron trifluoride pressure over the reaction mixture. The promoter can be mixed with the olefin feed and the reaction can be carried out in a batch or continuous process at temperatures of about 0° to 200° C and pressures ranging from atmospheric up to, for example, 6990 kPa (1,000 psig). The reaction temperature will change the oligomer distribution, with increasing temperatures favoring the production of dimers. Preferred reaction temperatures and pressures are about 20° to 90° C and 131 to 786 kPa (5 to 100 psig).

When a desired oligomer distribution is reached in the batch mode, the reaction is terminated by venting off excess boron trifluoride gas and purging with nitrogen gas to replace all boron trifluoride gaseous residue. The reaction product, unreacted monomer, and boron trifluoride-promoter complex residue are removed from the reactor for further processing. In the continuous mode the dissolved boron trifluoride may be degassed. The boron trifluoride-promoter complex may be separated by settling or coalescing from the reaction product.

The crude reactor product is then washed with an aqueous caustic solution and followed by one or more water washes to ensure neutralization.

The oligomer mixture from the reaction contains monomer, which can be removed by distillation. The monomer has been found to contain appreciable amounts of less reactive, isomerized material. However, this monomer can be recycled because it will react to form oligomers in the presence of fresh α-olefin monomer. The product mixture can be further separated by distillation to provide one or more product fractions having the desired viscosities for use in various lubricant applications such as avionics coolants, dielectric fluids, heat transfer fluids, drilling, hydraulic or metal working fluids, gear oils and crankcase lubricants.

The oligomer product can be hydrogenated by conventional methods to increase the oxidation stability of the product. Supported nickel catalysts are useful. For example, nickel on a Kieselguhr support gives good results. Batch or continuous processes can be used. For example, the catalyst can be added to the liquid and stirred under hydrogen pressure or the liquid may be trickled through a fixed bed of the supported catalyst under hydrogen pressure. Hydrogen pressures of about 786 to 6990 kPa (100 to 1,000 psig) at temperatures of about 150° to 300° C are especially useful. Preferably, the hydrogen pressure is from 2850 to 6990 kPa (400 to 1,000 psig) and the maximum temperature is 200° C to 300° C.

The amount of paraffin present in the product may be limited by preventing cracked fragments from being recycled to the reactor and eventually ending up in the dimer fraction. Typically, unreacted monomers and dimers are recycled to the reactor to increase the process efficiency or to produce a heavier oligomer distribution. Thermal degradation in the separational stage(s) or hydrocracking in the hydrogenator can result in cracking of higher molecular weight oligomers. These cracked fragments can migrate into the product stream. Generally, hydrocracking begins to occur with some severity at temperatures above 300° C, but may even occur to a lesser degree at lower temperatures. For example, in the production of a C₁₀ dimer, the cracked fragments in a molecular weight range from C₁₇ to C₂₂ will normally appear in the dimer fraction. Upon hydrogenation, some of the cracked fragments will become straight chain saturated hydrocarbons which can adversely affect the low temperature viscosity. The presence of the cracked fragments is supported by the fact, as discussed in Example 5, the crystals are composed of C₁₇ to C₂₂ hydrocarbons. Odd-carbon numbers cannot be synthesized from 1-decene oligomerization. Even-carbon numbers other than C₂₀ are also unlikely to be formed from 1-decene oligomerization. Therefore, carbon numbers other than C₂₀ are most likely formed from cracking of higher molecular weight oligomers.

The reaction conditions that are chosen may also affect the levels of the straight chain C₂₀ compounds. Favorable conditions may be obtained by selecting promoters such as hydroxy ketones, alcohol alkoxylates and alcohols as opposed to unfavorable promoters such as peroxides or SiO₂•12WO₃. Shorter residence times (less than 90 minutes, preferably less than 45 minutes) and lower reaction temperatures also favor lower amounts of the normal paraffins and desirable low temperature viscosities. Finally, in order to achieve reduced amounts of cracked fragments and subsequent normal paraffins, the dimer recycle should be eliminated.

### HYDROXY CARBONYL PROMOTER

By "hydroxy carbonyl," we mean an organic compound having both a hydroxyl group (containing an -OH unit) and a carbonyl group (either ketone or aldehydes).

Preferably, the hydroxy carbonyl is a hydroxy ketone because of concerns of unavailability, odor, and instability of hydroxy aldehydes. Preferably the hydroxyl group is either methyl hydroxyl, ethyl hydroxyl, propyl hydroxyl, butyl hydroxyl, pentyl hydroxyl, or hexyl hydroxyl, with the alkyl group being either straight or branched. Preferably the ketone group is either methyl ketone or ethyl ketone.

The hydroxy carbonyl can have more than one hydroxyl group and can have more than one carbonyl group. The compounds can have alternating hydroxyl and carbonyl groups (e.g., 4-hydroxy-4-methyl-2,6-heptanedione), or similar groups can be grouped together (e.g., 6-hydroxy-6-methyl-2,4-heptanedione). Neither the hydroxyl groups nor the carbonyl groups have to be the same throughout the molecule. For instance, in a dione compound, the carbonyl groups could be both methyl-ketone and ethyl-ketone (e.g., 4-hydroxy-4-methyl-2,6-octanedione).

More than one hydroxy ketone can be used. For instance, in one preferred embodiment, 1-hydroxy-2-butanone and 4-hydroxy-4-methyl-2-pentanone are used together.

The ratio of hydroxyl groups to carbonyl groups is preferably 1:1, (e.g., 4-hydroxy-4-methyl-2-pentanone). That ratio can be higher or lower. For instance, other hydroxy ketones conceived to be part of this invention include 2,6-dihydroxy-2,6-dimethyl-4-heptanone (ratio of 2:1) and 4-hydroxy-4-methyl-2,6-heptanedione (ratio of 1:2).

Suitable hydroxy ketones include, but are not limited to, hydroxy acetone, 1-hydroxy-2-butanone, 3-hydroxy-2-butanone, 4-hydroxy-2-butanone, 3-hydroxy-3-methyl-2-butanone, 3-hydroxy-2-pentanone, 4-hydroxy-2-pentanone, 1-hydroxy-3-pentanone, 2-hydroxy-3-pentanone, 3-hydroxy-3-methyl-2-pentanone, 3-hydroxy-4-methyl-2-pentanone, 4-hydroxy-3-methyl-2-pentanone, 4-hydroxy-4-methyl-2-pentanone, 2-hydroxy-2-methyl-3-pentanone, 3-hydroxy-2-hexanone, 4-hydroxy-2-hexanone, 4-hydroxy-3-hexanone, 5-hydroxy-3-hexanone, 4-hydroxy-4-methyl-3-hexanone, 4-hydroxy-5-methyl-3-hexanone, 5-hydroxy-4-methyl-3-hexanone, 5-hydroxy-5-methyl-3-hexanone, 4-hydroxy-3-heptanone, and 5-hydroxy-3-heptanone.

Preferably, the hydroxyl group is attached to a tertiary carbon, such as in 3-hydroxy-3-methyl-2-butanone, 3-hydroxy-3-methyl-2-pentanone, 4-hydroxy-4-methyl-2-pentanone, 2-hydroxy-2-methyl-3-pentanone, 4-hydroxy-4-methyl-3-hexanone, 5-hydroxy-5-methyl-3-hexanone. Of these hydroxy ketones, the preferred hydroxy ketones are β-hydroxy ketones, such as 4-hydroxy-4-methyl-2-pentanone and 5-hydroxy-5-methyl-3-hexanone.

A preferred hydroxy carbonyl is 4-hydroxy-4-methyl-2-pentanone because it gives no emulsion in the reactor or in the water wash, as discussed below. 1-hydroxy-2-butanone gives a slight emulsion in the water wash and hydroxy acetone gives a significant emulsion in the water wash. In general, we have found that tertiary hydroxy ketones, such as, 4-hydroxy-4-methyl-2-pentanone and 3-hydroxy-3-methyl-2-butanone form no, or appreciably lesser amounts of, emulsion than secondary or primary hydroxy ketones. Secondary hydroxy ketones, such as, 3-hydroxy-2-butanone appear to form more dimer than the corresponding primary hydroxy ketones, such as, 1-hydroxy-2-butanone. Also, higher conversion rates have been observed with α-hydroxy ketones versus β-hydroxy ketones.

### SECONDARY PROMOTERS

In one embodiment, a second promoter can be used in conjunction with the boron trifluoride and the primary promoter. Possible secondary promoters include aldehydes, alcohols, alcohol alkoxylates, carboxylic acids, ethers, ketones, and their mixtures. These secondary promoters are used to further initiate oligomerization.

Preferably an alcohol, such as methanol, is used as a secondary promoter to achieve a faster reaction rate. The amount of alcohol or alcohol alkoxylate used depends, in part, on the ratio of hydroxyl groups to carbonyl groups in the hydroxy carbonyl. Less secondary promoter is needed if this ratio is high (i.e., if there is an excess of hydroxyl groups to carbonyl groups).

Alcohol alkoxylates useful as secondary promoters can be represented, for example, by the formula:

RO-(CHR'-CHR"-O)n H

where R is hydrocarbyl containing from 1 to 24 carbons, including mixtures thereof, R' and R" are independently hydrogen, methyl, or ethyl, and n averages 1 to 15. Such alcohol alkoxylates are disclosed in U.S. Patent No. 5,068,487, entitled "Olefin Oligomerization With BF3 Alcohol Alkoxylate Co-Catalysts,"

A ketone, such as methyl-ethyl-ketone, can also be used as a secondary promoter to suppress the formation of higher oligomers.

### EXAMPLES

The invention will be further illustrated by the following examples, which set forth particularly advantageous method embodiments. While the Examples are provided to illustrate the present invention, they are not intended to limit it.

### Gas Chromatography Method for Oligomer Distribution

Hewlett-Packard Model 5890 series II gas chromatograph was used to analyze oligomer distribution in all the examples presented. The instrument had a Chrompack Ultimetal HT SimDist CB 10 m x 0.5 mm ID column with a 0.15 µm stationary phase film thickness. The instrument was set up in the following oven temperature profile:
Initial temperature - 60° C
Ramp up rate - 16° C per minute
Final temperature - 435° C
Final time - 12 minutes
Carrier - Helium
Flow rate - 5 ml/min
Flame Ionization Detector
Cool on-column injection 0.2 µl injection volume

### Gas Chromatography Method for Percent Paraffin

Hewlett-Packard Model 5890 series II gas chromatograph was used to analyze paraffin content in all the examples presented. The instrument had a 30 m x 0.25 mm ID column with a SE-30 0.25 µm stationary phase film thickness. The instrument was set up in the following oven temperature profile:
Initial temperature - 100° C
Initial time - 1 minute
Ramp up rate - 6° C per minute
Final temperature - 300° C
Final time - 10 minutes

### Example 1

### C₁₀ Dimer

The oligomerization reaction was carried out in either an autoclave reactor or a continuous reactor train. The autoclave reactor was equipped with a packless stirrer, and all wetted surfaces were made of 316 stainless steel. The reactor had an external electrical heater and an internal cooling coil for temperature control. The reactor was equipped with a dip tube, gas inlet and vent valves, and a pressure relief rupture disc. Prior to the monomer charge, the reactor was cleaned, purged with nitrogen and tested for leaks.

One thousand grams of 1-decene was charged into the batch reactor. The promoter, acetol, was added to a concentration of 0.25 wt.% based on feed. The entire reactor content was heated under nitrogen blanket to reach 60° C. When the reactor temperature reached equilibrium, the reactor was then evacuated to remove the nitrogen. Boron trifluoride gas was then sparged slowly with agitation in addition to temperature control via a cooling coil to avoid reactor temperature overrun. Additional boron trifluoride was added as necessary to maintain a reactor pressure of 303 kPa (30 psig). The reaction was terminated after two hours by venting off excess boron trifluoride gas and purging with nitrogen. The reaction product was then washed with a 4 wt.% aqueous sodium hydroxide solution followed by several water washes to ensure neutralization. The product was saved for further treatments such as hydrogenation and fractionation. The resultant dimer fraction generally had a 97+% purity based on the gas chromatography method for oligomer distribution.

The continuous monomer feed reactor was equipped with monomer, promoter and gas inlet ports, vent valves, and a pressure relief rupture disc. Prior to start-up, the reactor was cleaned, purged with nitrogen and tested for leaks. A 1-decene monomer flow rate, chosen in the range of 700-2300 grams per hour, a reactor temperature of 75° C and a reactor pressure of 30 psig were controlled throughout the reaction period. The reactor had a BF₃ gas cap and its liquid volume was controlled through a level control device at approximately one half of the reactor volume. The promoter, n-butanol or denatured ethanol, was added to a concentration of 0.25 wt.% based on feed. The reaction product was discharged to a low pressure flash tank to remove the gaseous material. The liquid product stream was then subjected to the neutralization and washing steps. The product was further treated by hydrogenation and fractionation. The resulting dimer fraction generally had a 97+% purity based on the gas chromatography method for oligomer distribution. A mixture of the dimer fractions from both the batch and the continuous reactor systems was used as a control and for the spiking study.

### Example 2

### C₈ Dimer

A reaction system with continuous monomer, 1-octene, feed and two-reactors in series was used in this example. The mechanical setup of both reactors was similar as described in Example 1. The effluent from the first reactor was introduced to the second. The second reactor was hydraulicly full. Temperature of both reactors were controlled at 50° C and the pressure of the first reactor was controlled at 372 kPa (40 psig). Due to the pressure drop between the two reactors, the pressure in the second reactor was slightly lower than that of the first, generally less than a 1 psig differential. The promoters, methanol and 4-hydroxy-4-methyl-2-pentanone, were added continuously to the first reactor at a concentration of 0.1 wt.% and 0.65 wt.% respectively, based on feed. The reaction product was discharged to a low pressure flash tank to remove the gaseous material. The liquid product stream was then subjected to neutralization and washing steps. The product was further treated by hydrogenation and fractionation. The resultant dimer fraction generally had a 97+% purity based on the gas chromatography method for oligomer distribution. A mixture of the dimer fractions from the continuous reactor system was used as a control and for the spiking study.

### Example 3

### C₁₂ Dimer

The dimer sample was obtained from the operation of two large continuous stirred tank reactors in series. The monomer feed contained 98% 1-dodecene and 2% 1-decene . The reactor pressure was controlled at 303 kPa (30 psig) and the reactor temperature at 32° C. The reaction product and unreacted monomer were separated by distillation. The dimer-rich fraction from the distillation was hydrogenated. After saturation, a sample of the dimer-rich fraction was further fractionated to obtain 97+% purity.

### Example 4

### C₁₄ Dimer

The oligomerization reaction was carried out in an autoclave reactor as described in Example 1. Two batch reactions were made to produce oligomer samples. In the first run, five-hundred grams of 1-tetradecene was charged into the reactor. The promoters, methanol and 4-hydroxy-4-methyl-2 pentanone, were added to a concentration of 0.05 wt.% and 0.25 wt.% of feed, respectively. The entire reactor was heated under a nitrogen blanket to reach 75° C. When the reactor temperature reached equilibrium, the reactor was then evacuated to remove the nitrogen. Boron trifluoride gas was then sparged slowly with agitation and temperature control via a cooling coil to avoid reactor temperature overrun. Additional boron trifluoride was added as necessary to maintain a reactor pressure of 234 kPa (20 psig). The reaction was terminated after two hours by venting off excess boron trifluoride gas and purging with nitrogen. The reaction product was then washed with a 4 wt.% aqueous sodium hydroxide solution followed by several water washes to ensure neutralization. In the second run, one-thousand grams of 1-tetradecene was charged into the reactor. The promoter, acetol, was added to a concentration of 0.125 wt.% of feed. Other operating conditions were identical to those cited in the first run except for the reactor temperature, which was maintained at 60° C, and the reactor pressure, which was 303 kPa (30 psig). The reaction products from the two runs were combined. The resultant product was further treated by hydrogenation and fractionation. The resultant dimer fraction generally had a 97+% purity.

### Example 5

### Formation and Filtration of Crystals

A sample of relatively poor quality decene based hydrogenated dimer, as characterized by a -54° C of 1784 cSt, was placed into a temperature controlled bath at -30° C. Shortly after placing the sample into the bath a haze developed; after 24 hours crystalline material was observed. The crystals were allowed to continue to grow over a period of two weeks. However, it was observed that the crystals would quickly disappear if removed from the -30° C bath. Subsequently, we discovered that the crystals were dissolved by the bulk fluid as it warmed.

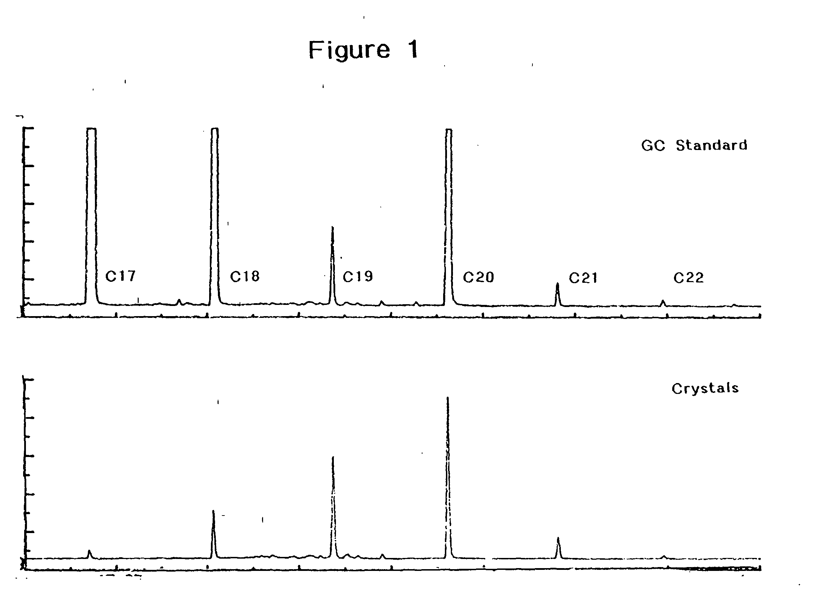

The sample was placed into a glove box that was temperature controlled to -45° C in order to isolate the crystals for identification while preventing their dissolution. The glove box allowed the crystals to be vacuum filtered and washed with hexane in the cold environment. The apparatus and materials were allowed to achieve temperature equilibrium within the glove box at -45° C. Subsequently, gas chromatographic analysis was performed on the crystals which once isolated were stable and did not quickly melt at ambient temperatures. The gas chromatographic analysis revealed, upon comparison to standards (see Figure 1), that the crystals eluted identically to normal paraffin standards which have a molecular weight corresponding to hydrocarbons having from 17 to 22 carbon atoms in length. The predominant component was identified as normal eicosane (20 carbon atoms).

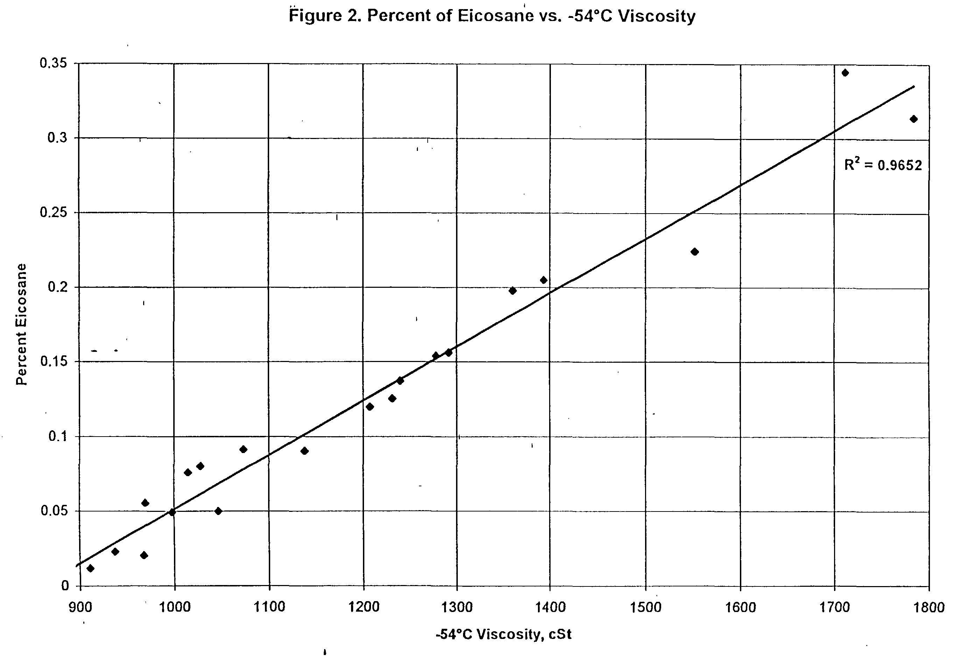

Several samples were analyzed by gas chromatography and their -54° C kinematic viscosity measured. These samples consisted of 1-decene hydrogenated dimers prepared both from batch and continuous modes of formation. The results from the gas chromatographic analysis and the -54° C kinematic viscosity are plotted in Figure 2. The -54° C viscosity increases proportionally to the concentration of the normal eicosane, even at very low concentrations.

Various amounts of pure normal paraffins were added to hydrogenated dimers of 1-octene, 1-decene, 1-dodecene and 1-tetradecene, separately. Each paraffin was added to the corresponding hydrogenated dimer, i.e. eicosane to the 1-decene hydrogenated dimer and tetracosane to the 1-dodecene hydrogenated dimer.

### Example 6

### Spiking of 1-Octene Dimers

Table 1 shows the results from adding various amounts of hexadecane to the 1-octene hydrogenated dimer. The samples were examined by gas chromatographic analysis for the percent n-paraffin and the -54° C kinematic viscosity measured. The difference between the analysis and the amount added is due to the amount of normal paraffin present in the sample before any addition.

**Table 1.**

| Spiking Study for 1-Octene Hydrogenated Dimer | | |
|---|---|---|
| -54° C Viscosity, cSt | Amount of n-Hexadecane Added, % | Total n-Hexadecane by GC, % |
| 245 | 0.0 | 0.04 |
| 243 | 0.1 | 0.14 |
| 260 | 0.4 | 0.46 |
| 399 | 1.0 | 1.06 |

### Example 7

### Spiking of 1-Decene Dimers

Example 6 was repeated except n-eicosane and 9-methylnonadecane were used for spiking the 1-decene hydrogenated dimers. The samples were analyzed by gas chromatography for the percentages of the n-paraffin and the -40° C and -54° C kinematic viscosities were measured. The results are shown in Tables 2 and 3 for the n-eicosane and 9-methylnonadecane spiking, respectively.

**Table 2.**

| Spiking of 1-Decene Hydrogenated Dimer with n-Eicosane | | | |
|---|---|---|---|
| -54° C Viscosity, cSt | -40° C Viscosity, cSt | Amount of n-Eicosane Added, % | Total n-Eicosane by GC, % |
| 975 | 227 | 0.0 | 0.017 |
| 1253 | 231 | 0.1 | 0.115 |
| 2898 | 404 | 1.0 | 1.030 |

**Table 3.**

| Spiking of 1-Decene Hydrogenated Dimer with 9-Methylnonadecane | | | |
|---|---|---|---|
| -54° C Viscosity, cSt | -40° C Viscosity, cSt | Amount of 9-Methylnonadecane Added, % | Total 9-Methylnonadecane by GC, % |
| 975 | 227 | 0.0 | 0.518 |
| 971 | 230 | 0.1 | 0.609 |
| 1055 | 228 | 1.0 | 1.440 |

### Example 8

### Spiking of 1-Dodecene Dimers

Example 6 was repeated except n-tetracosane was used for spiking the 1-dodecene hydrogenated dimers. The samples were analyzed by gas chromatography for the percent n-paraffin and the -40° C kinematic viscosity measured. The results are shown in Table 4.

**Table 4.**

| Spiking Study for 1-Dodecene Hydrogenated Dimer | | |
|---|---|---|
| -40° C Viscosity, cSt | Amount of n-Tetracosane Added, % | Total n-Tetracosane by GC, % |
| 925 | 0.0 | 0.26 |
| 950 | 0.1 | 0.38 |
| 1567 | 0.4 | 0.66 |
| no flow | 1.0 | 1.31 |

### Example 9

### Spiking of 1-Tetradecene Dimers

Example 6 was repeated except n-octacosane was used for spiking the 1-tetradecene hydrogenated dimers. The samples-were analyzed by gas chromatography for the percent n-paraffin and the -20° C kinematic viscosity measured. The results are shown in Table 5.

**Table 5.**

| Spiking Study for 1-Tetradecene Hydrogenated Dimer | | |
|---|---|---|
| -20° C Viscosity, cSt | Amount of n-Octacosane Added, % | Total n-Octacosane by GC, % |
| 342 | 0.0 | 0.05 |
| 3177 | 0.1 | 0.16 |

### Example 10

### Residence Time Effects on Dimer Quality

The samples were prepared as in Example 1 except the promoter was ethanol. The residence time was varied and listed in Table 6. The amount of n-eicosane was determined by gas chromatography and the -54° C kinematic viscosity was measured.

**Table 6.**

| Residence Time Effect on n-Eicosane Content | | |
|---|---|---|
| Residence Time, min | -54° C Viscosity, cSt | n-Eicosane, % |
| 30 | 911 | 0.012 |
| 45 | 1047 | 0.050 |

The examples show that surprisingly small amounts of the respective normal paraffins can greatly affect the low temperature viscosities of the hydrogenated dimers. Generally, it had been previously believed that normal paraffins did not exist in the hydrogenated olefin oligomers. In fact, methyl branched isomers were previously identified to deleteriously effect the low temperature viscosities. While this is true, we have found that the effect of the normal paraffins is significantly greater, overshadowing the effect of the methyl paraffins. Hence, it is of the utmost importance to limit the concentration of n-paraffins in the hydrogenated dimer products.

In the process of investigating this invention, we found one hydrogenated 1-decene dimer from a third party which did have a -54° C viscosity of 970 cSt and 0.056% n-eicosane. This sample appears to be the only one of its kind and we have no indication that this sample has been or could be reproduced. Until now, the production of routinely good quality 1-decene hydrogenated dimer has not been controllable. Now that we have discovered the true cause of poor low temperature performance, we are able to routinely produce hydrogenated dimers that have superior low temperature performance.

## Claims

1. A hydrogenated dimer composition comprising at least one dimer selected from the group consisting of:
(a) 1-octene dimer having less than 0.50 wt.% normal hexadecane, and having a -54° C viscosity of less than 280 cSt;
(b) 1-decene dimer having less than 0.05 wt.% normal eicosane, and having a -54° C viscosity of less than 1000 cSt and a -40° C viscosity of less than 240 cSt;
(c) 1-dodecene dimer having less than 0.60 wt.% normal tetracosane, and having a -40° C viscosity of less than 1000 cSt; and
(d) 1-tetradecene dimer having less than 0.10 wt.% normal octacosane, and having a -20° C viscosity of less than 1000 cSt.

2. A hydrogenated dimer composition according to Claim 1 comprising 1-octene dimer having less than 0.1 wt.% normal hexadecane, and having a -54° C viscosity of less than 250 cSt.

3. A hydrogenated dimer composition according to Claim 1 comprising 1-decene dimer having from 0.02 wt.% to 0.05 wt.% normal eicosane, and having a -54° C viscosity of less than 1000 cSt and a -40° C viscosity of less than 240 cSt.

4. A hydrogenated dimer composition according to Claim 3 wherein the 1-decene dimer has less than 0.03 wt.% normal eicosane, and having a -54° C viscosity of less than 980 cSt and a -40° C viscosity of less than 230 cSt.

5. A dimer composition according to Claim 1 comprising 1-dodecene dimer having less than 0.40 wt.% normal tetracosane, and having a -40° C viscosity of less than 950 cSt.

6. A dimer composition according to Claim 5 comprising 1-dodecene dimer having less than 0.30 wt.% normal tetracosane.

7. A dimer composition according to Claim 1 wherein the 1-tetradecene dimer has less than 0.06 wt.% normal tetracosane, and having a -20° C viscosity of less than 500 cSt.

8. A process for producing a hydrogenated dimer composition comprising the steps of:
(a) reacting an olefinic monomer to produce a dimer composition comprising dimer and higher oligomers, wherein the process to produce the dimer composition is carried out at a temperature of from 0° C to 200° C and a pressure of from atmospheric to 6990 kPa (1000 psig), and wherein the dimer composition has at least one dimer selected from the group consisting of:
(1) 1-octene dimer having less than 0.50 wt.% normal C₁₆ materials;
(2) 1-decene dimer having less than 0.05 wt.% normal C₂₀ materials;
(3) 1-dodecene dimer having less than 0.60 wt.% normal C₂₂ materials; and
(4) 1-tetradecene dimer having less than 0.10 wt.% normal C₂₄ materials;
(b) hydrogenating the dimer composition to produce a hydrogenated dimer composition, wherein the hydrogenation process is carried out at a maximum temperature of from 150° C to 300° C and a hydrogen pressure of from 786 to 6990 kPa (100 to 1000 psig), and wherein the hydrogenated dimer composition has at least one dimer selected from the group consisting of:
(1) 1-octene dimer having less than 0.50 wt.% normal hexadecane, and having a -54° C viscosity of less than 280 cSt;
(2) 1-decene dimer having less than 0.05 wt.% normal eicosane, and having a -54° C viscosity of less than 1000 cSt and a -40° C viscosity of less than 240 cSt;
(3) 1-dodecene dimer having less than 0.60 wt.% normal tetracosane, and having a -40° C viscosity of less than 1000 cSt; and
(4) 1-tetradecene dimer having less than 0.10 wt.% normal octacosane, and having a -20° C viscosity of less than 1000 cSt.

9. A process for producing a hydrogenated dimer composition according to Claim 8 wherein the process to produce the dimer composition is carried out at a temperature of from 20° C to 90° C and a pressure of from 131 to 786 kPa (5 to 100 psig), wherein the hydrogenation process is carried out at a maximum temperature of from 200° C to 300° C and a hydrogen pressure of from 2850 to 6990 kPa (400 to 1000 psig), and wherein the process further comprising the step of separating higher oligomers from the hydrogenated dimer composition.

10. A process for producing a hydrogenated dimer composition according to claim 8, wherein:
i) the process to produce the dimer composition is carried out at a temperature of from 20°C to 90°C and a pressure of from 131 to 786 kPa (5 to 100 psig) ;
ii) the higher oligomers are separated from the reaction mixture prior to hydrogenation; and
iii) the dimer composition is hydrogenated at a maximum temperature of from 200°C to 300°C and a hydrogen pressure of from 2850 to 6990 kPa (400 to 1000 psig).

## Revendications

1. Composition de dimère hydrogéné comprenant au moins un dimère choisi parmi le groupe constitué du :
(a) dimère de l'oct-1-ène contenant moins de 0,50 % en poids d'hexadécane normal, et ayant une viscosité à -54 °C inférieure à 280 cSt ;
(b) dimère du déc-1-ène contenant moins de 0,05 % en poids d'eicosane normal, et ayant une viscosité à -54 °C inférieure à 1000 cSt et une viscosité à -40 °C inférieure à 240 est ;
(c) dimère de dodéc-1-ène contenant moins de 0,60 % en poids de tétracosane normal, et ayant une viscosité à -40 °C inférieure à 1000 cSt ; et
(d) dimère de tétradéc-1-ène contenant moins de 0,10 % en poids d'octacosane normal, et ayant une viscosité à -20 °C inférieure à 1000 cSt.

2. Composition de dimère hydrogéné selon la revendication 1 comprenant un dimère de oct-1-ène contenant moins de 0,1 % en poids d'hexadécane normal, et ayant une viscosité à -54 °C inférieure à 250 est ;

3. Composition de dimère hydrogéné selon la revendication 1 comprenant un dimère de déc-1-ène contenant moins de 0,02 % à 0,05 % en poids d'eicosane normal, et ayant une viscosité à -54 °C inférieure à 1000 cSt et une viscosité à -40 °C inférieure à 240 cSt.

4. Composition de dimère hydrogéné selon la revendication 3 comprenant un dimère de déc-1-ène contenant moins de 0,03 % en poids d'eicosane normal, et ayant une viscosité à -54 °C inférieure à 980 cSt et une viscosité à -40 °C inférieure à 230 cSt.

5. Composition de dimère hydrogéné selon la revendication 1 comprenant un dimère de dodéc-1-ène contenant moins de 0,40 % en poids de tétracosane normal, et ayant une viscosité à -40 °C inférieure à 950 cSt.

6. Composition de dimère selon la revendication 5 comprenant un dimère de dodéc-1-ène contenant moins de 0,30 % en poids de tétracosane normal.

7. Composition de dimère selon la revendication 1, dans laquelle un dimère de tétradéc-1-ène contient moins de 0,06 % en poids de tétracosane normal, et ayant une viscosité à -20 °C inférieure à 500 cSt.

8. Procédé pour la production d'une composition de dimère hydrogéné comprenant les étapes :
(a) de réaction d'un monomère oléfinique pour produire une composition de dimères comprenant un dimère et des oligomères supérieurs, dans laquelle le procédé de production de la composition de dimère a été réalisé à une température variant de 0 °C à 200 °C et à une pression allant de la pression atmosphérique jusqu'à 6990 kPa (1000 psig), et dans laquelle la composition de dimère possède au moins un dimère choisi par le groupe constitué du :
(1) dimère de l'oct-1-ène contenant moins de 0,50 % en poids de matériaux normaux C₁₆ ;
(2) dimère du déc-1-ène contenant moins de 0,05 % en poids de matériaux normaux C₂₀ ;
(3) dimère du dodéc-1-ène contenant moins de 0,60 % en poids de matériaux normaux C₂₂ ; et
(4) dimère du tétradéc-1-ène contenant moins de 0,10 % en poids de matériaux normaux C₂₄ ;
(b) d'hydrogénation de la composition de dimères pour produire une composition de dimères hydrogénés, dans laquelle le procédé d'hydrogénation a été effectué à une température maximale de 150 °C à 300 °C et à une pression d'hydrogène de 786 à 6990 kPa (100 à 1000 psig), et dans laquelle la composition de dimères hydrogénés possède au moins un dimère choisi parmi le groupe constitué du :
(1) dimère du déc-1-ène contenant moins de 0,50 % en poids d'hexadécane normal, et ayant une viscosité à -54 °C inférieure à 280 cSt ;
(2) dimère du déc-1-ène contenant moins de 0,05 % en poids d'eicosane normal, et ayant une viscosité à -54 °C inférieure à 1000 cSt et une viscosité à -40 °C inférieure à 240 cSt ;
(3) dimère du dodéc-1-ène contenant moins de 0,60 % en poids de tétracosane normal, et ayant une viscosité à -40 °C inférieure à 1000 cSt ; et
(4) dimère tétradéc-1-ène contenant moins de 0,10 % en poids d'octacosane normal, et ayant une viscosité à -20 °C inférieure à 1000 cSt.

9. Procédé pour la production d'une composition de dimères hydrogénés selon la revendication 8, dans lequel le procédé de production de la composition de dimères a été effectué à une température de 20 °C à 90 °C et à une pression de 131 à 786 kPa (5 à 100 psig), dans lequel le procédé d'hydrogénation a été effectué à une température maximale de 200 °C à 300 °C et à une pression d'hydrogène de 2850 à 6990 kPa (400 à 1000 psig), et dans lequel le procédé comprend en outre l'étape de séparation des oligomères supérieurs de la composition de dimères hydrogénés.

10. Procédé pour la production d'une composition de dimères hydrogénés selon la revendication 8, dans laquelle :
i) le procédé pour la production de la composition de dimères a été effectué à une température de 20 °C à 90 °C et à une pression de 131 à 786 kPa (5 à 100 psig) ;
ii) les oligomères supérieurs sont séparés du mélange réactionnel avant l'hydrogénation ; et
iii) la composition de dimères a été hydrogénée à une température maximale de 200 °C à 300 °C et à une pression d'hydrogène de 2850 à 6990 kPa (400 à 1000 psig).

## Patentansprüche

1. Hydrierte Dimer-Zusammensetzung, aufweisend mindestens ein Dimer, ausgewählt aus der Gruppe, bestehend aus:
(a) 1-Octen-Dimer mit weniger als 0,50 Gew.% n-Hexadecan und mit einer Viskosität bei -54°C von weniger als 280 cSt;
(b) 1-Decen-Dimer mit weniger als 0,05 Gew.% n-Eicosan und mit einer Viskosität bei -54°C von weniger als 1.000 cSt und einer Viskosität bei -40°C von weniger als 240 cSt;
(c) 1-Dodecen-Dimer mit weniger als 0,60 Gew.% n-Tetracosan und mit einer Viskosität bei -40°C von weniger als 1.000 cSt;
(d) 1-Tetradecen-Dimer mit weniger als 0,10 Gew.% n-Octacosan und mit einer Viskosität bei -20°C von weniger als 1.000 cSt.

2. Hydrierte Dimer-Zusammensetzung nach Anspruch 1, aufweisend 1-Octen-Dimer mit weniger als 0,1 Gew.% n-Hexadecan und mit einer Viskosität bei -54°C von weniger als 250 cSt.

3. Hydrierte Dimer-Zusammensetzung nach Anspruch 1, aufweisend 1-Decen-Dimer mit 0,02% bis 0,05 Gew.% n-Eicosan und mit einer Viskosität bei -54°C von weniger als 1.000 cSt und mit einer Viskosität bei -40°C von weniger als 240 cSt.

4. Hydrierte Dimer-Zusammensetzung nach Anspruch 3, worin das 1-Decen-Dimer weniger als 0,03 Gew.% n-Eicosan hat und eine Viskosität bei -54°C von weniger als 980 cSt und eine Viskosität bei -40°C von weniger als 230 cSt hat.

5. Dimer-Zusammensetzung nach Anspruch 1, aufweisend 1-Dodecen-Dimer mit weniger als 0,40 Gew.% n-Tetracosan und mit einer Viskosität bei -40°C von weniger als 950 cSt.

6. Dimer-Zusammensetzung nach Anspruch 5, aufweisend 1-Dodecen-Dimer mit weniger als 0,30 Gew.% n-Tetracosan.

7. Dimer-Zusammensetzung nach Anspruch 1, bei welcher das 1-Tetradecen-Dimer weniger als 0,06 Gew.% n-Tetracosan aufweist und eine Viskosität bei -20°C von weniger als 500 cSt hat.

8. Verfahren zum Herstellen einer hydrierten Dimer-Zusammensetzung, umfassend die Schritte:
(a) Umsetzen eines olefinischen Monomers, um eine Dimer-Zusammensetzung zu erzeugen, aufweisend Dimer und höhere Oligomere, wobei das Verfahren zum Herstellen der Dimer-Zusammensetzung ausgeführt wird bei einer Temperatur von 0°C bis 200°C und einem Druck von Atmosphärendruck bis 6.990 kPa (1.000 psig) und wobei die Dimer-Zusammensetzung mindestens ein Dimer hat, ausgewählt aus der Gruppe, bestehend aus:
(1) 1-Octen-Dimer mit weniger als 0,50 Gew.% n-C₁₆-Substanzen;
(2) 1-Decen-Dimer mit weniger als 0,05 Gew.% n-C₂₀-Substanzen;
(3) 1-Dodecen-Dimer mit weniger als 0,60 Gew.% n-C₂₂-Substanzen; und
(4) 1-Tetradecen-Dimer mit weniger als0,10 Gew.% n-C₂₄-Substanzen;
(b) Hydrieren der Dimer-Zusammensetzung, um eine hydrierte Dimer-Zusammensetzung zu erzeugen, wobei der Hydrierungsprozess ausgeführt wird bei einer höchsten Temperatur von 150° bis 300°C und einem Wasserstoff-Druck von 786 bis 6.990 kPa (100 bis 1.000 psig) und worin die hydrierte Dimer-Zusammensetzung mindestens ein Dimer hat, ausgewählt aus der Gruppe, bestehend aus:
(1) 1-Octen-Dimer mit weniger als 0,50 Gew.% n-Hexadecan und mit einer Viskosität bei -54°C von weniger als 280 cSt;
(2) 1-Decen-Dimer mit weniger als 0,50 Gew.% n-Eicosan und mit einer Viskosität bei -54°C von weniger als 1.000 cSt und mit einer Viskosität bei -40°C weniger als 240 cSt;
(3) 1-Dodecen-Dimer mit weniger als 0,60 Gew.% n-Tetracosan und mit einer Viskosität bei -40°C von weniger als 1.000 cSt; und
(4) 1-Tetradecen-Dimer mit weniger als 0,10 Gew.% n-Octacosan und mit einer Viskosität bei -20°C von weniger als 1.000 cSt.

9. Verfahren zum Herstellen einer hydrierten Dimer-Zusammensetzung nach Anspruch 8, wobei das Verfahren zum Herstellen der Dimer-Zusammensetzung ausgeführt wird bei einer Temperatur von 20°C bis 90°C und einem Druck von 131 bis 786 kPa (5 bis 100 psig) und wobei der Hydrierungsprozess ausgeführt wird bei einer höchsten Temperatur von 200° bis 300°C und einem Wasserstoff-Druck von 2.850 bis 6.990 kPa (400 bis 1.000 psig) und wobei der Prozess ferner die Schritte des Abtrennens höherer Oligomere von der hydrierten Dimer-Zusammensetzung umfasst.

10. Verfahren zum Herstellen einer hydrierten Dimer-Zusammensetzung nach Anspruch 8, worin:
i) das Verfahren zum Herstellen der Dimer-Zusammensetzung ausgeführt wird bei einer Temperatur von 20° bis 90°C und einem Druck von 131 kPa bis 786 kPa (5 bis 100 psig);
ii) die höheren Oligomere aus dem Reaktionsgemisch vor der Hydrierung abgetrennt werden; und
iii) die Dimer-Zusammensetzung bei einer höchsten Temperatur von 200° bis 300°C und einem Wasserstoff-Druck von 2.850 bis 6.990 kPa (400 bis 1.000 psig) hydriert wird.
